# EUROPEAN PATENT APPLICATION

(11) **EP 1 367 050 A1**
(43) Date of publication of application: **03.12.2003**
(21) Application number: 02705046.7
(22) Date of filing: 27.02.2002
(51) Int. Cl.: C07D 209/08

(54) **2-METHYLINDOLE-4-ACETIC ACID, PROCESS FOR PRODUCING THE SAME, AND PROCESS FOR PRODUCING INTERMEDIATE THEREFOR**

(30) Priority: 01.03.2001 JP 2001057165
(71) Applicant: ONO PHARMACEUTICAL CO., LTD., Osaka-shi, Osaka 541-8526 (JP)
(72) Inventor: HASEGAWA, Tomoyuki c/o Fukui Research Institute, Yamagishi Mikuni-cho Sakai-gun, Fukui (JP); KAWANAKA, Yasufumi c/o Fukui Research Institute, Yamagishi Mikuni-cho Sakai-gun, Fukui (JP)
(74) Representative: Henkel, Feiler, Hänzel
(86) International application number: JP0201793
(87) International publication number: WO02070477

(57) **Abstract**

2-Methylindole-4-acetic acid, which is represented by the formula (I) and is an intermediate for an important indole derivative as medicines; and a process for producing the compound and the synthetic intermediates.

The process for producing 2-methylindole-4-acetic acid and synthetic intermediates is described in this invention. As shown in formula (I), 2-methylindole-4-acetic acid is an intermediate of important indole derivative which is useful tools for medicine.

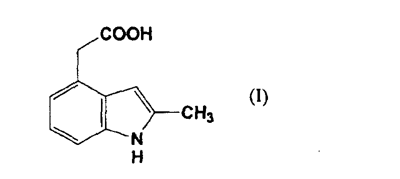

According to the process described in this invention, 2-methylindole-4-acetic acid, which is an intermediate of important indole derivative as medicines, can be produced in high yield using inexpensive reagents under mild conditions which are suitable for industrial production.

## Description

### TECHNICAL FIELD

The invention relates to 2-methylindole-4-acetic acid, process for producing the same, and process for producing intermediate therefor.

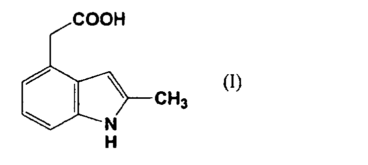

More specifically, the invention relates to 2-methylindole-4-acetic acid represented by formula (I), which is an intermediate of medicine: 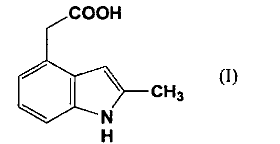 process for producing the same, and process for producing intermediate therefor.

### BACKGROUND ART

As shown in formula (I), 2-methylindole-4-acetic acid is an intermediate of indole derivatives, which is important for medicine, and it is a novel compound.

As a compound similar to the compound represented by the formula (I), for example, the following are known.
(1) A preparation of indole-4-acetic acid that is represented by a scheme: 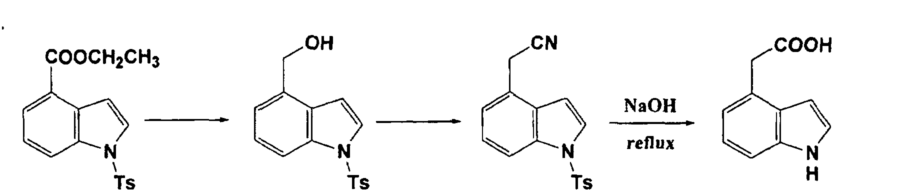 (wherein Ts represents tosyl group) has been published in J. Org. Chem., 44(22), 4003-4005 (1979).
(2) Also, the preparation of 1-tosylindole-4-acetic acid or 4-cyanomethyl-1-tosylindole that is represented by a scheme: 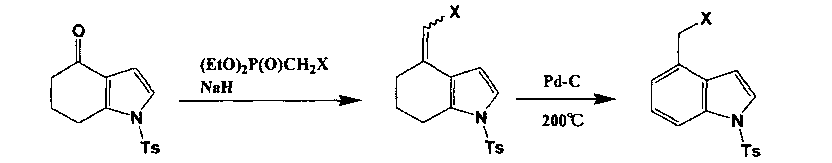 (wherein X represents CN or COOC₂H₅) has been published in Hererocycles, 24(9), 2611-2618 (1986).
   As a process for the preparation of 2-methyl-4-oxo-4,5,6,7-tetrahydroindole that is a intermediate of the compound represented by the formula (I) of the present invention,
(3) a process for the preparation represented by a scheme: 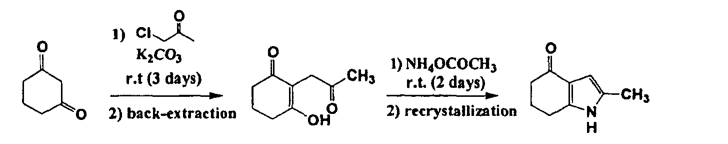 has been published in the specification of US 4868315,
(4) a process for the preparation represented by a scheme: 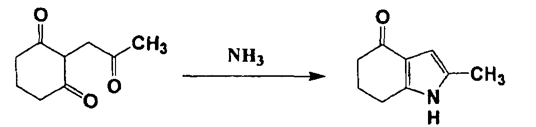 has been published in Justus Liebigs Ann. Chem., 655, 20-26 (1962), and
(5) a process for the preparation represented by a scheme: 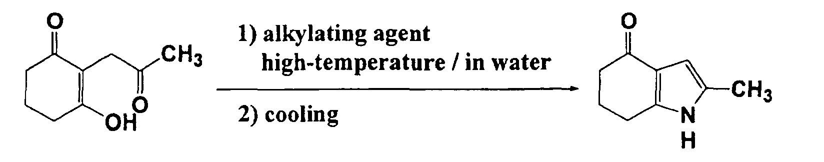 has been published in the specification of JP2-15058.
   Also, as a compound similar to 2-methyl-4-oxo-4,5,6,7-tetrahydroindole,
(6) a process for the preparation represented by a scheme: 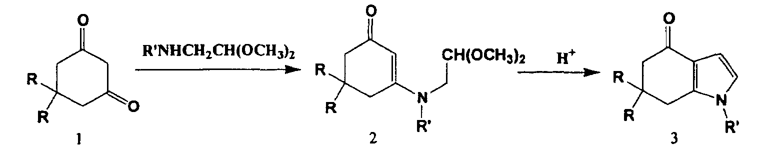 (wherein R represents H or CH₃, R' represents H, CH₃ or -CH₂-C₆H₅) has been published in J. Org. Chem., 43(18), 3541-3544 (1978).
   As a similar process of halogenation reaction that is one of the process of the compound represented by the formula (I) of the present invention,
(7) A process for the preparation of a halogen compound represented by a scheme: 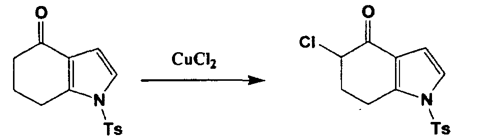 has been published in Hererocycles, 23(1), 165-170 (1985).

### DISCLOSURE OF THE INVENTION

The process for the preparation of 2-methylindole-4-acetic acid, which is an intermediate in medicine, under mild condition, using inexpensive reagents, and in high yield is desired. Especially, the process for the preparation of the compound industrially is desired.

The present inventors have energetically studied to find the process for the preparation of the 2-methylindole-4-acetic acid in high yield using inexpensive reagents under mild conditions suitable for industrial production. As a result, the novel process for the preparation represented by following scheme 1 was found out and the present invention was completed. 2-Methylindole-4-acetic acid represented by the formula (I) is a novel compound.

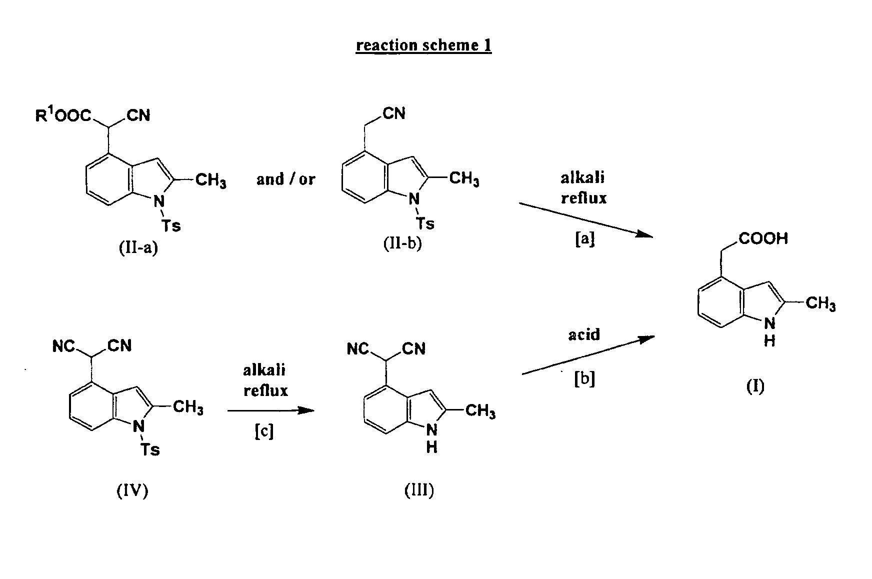

In the reaction scheme 1, R¹ represents C1-4 alkyl and Ts represents tosyl group.

For example, the compound represented by the formula (II-a), (II-b) and (IV) may be prepared according to the following reaction schemes 2 and 3.

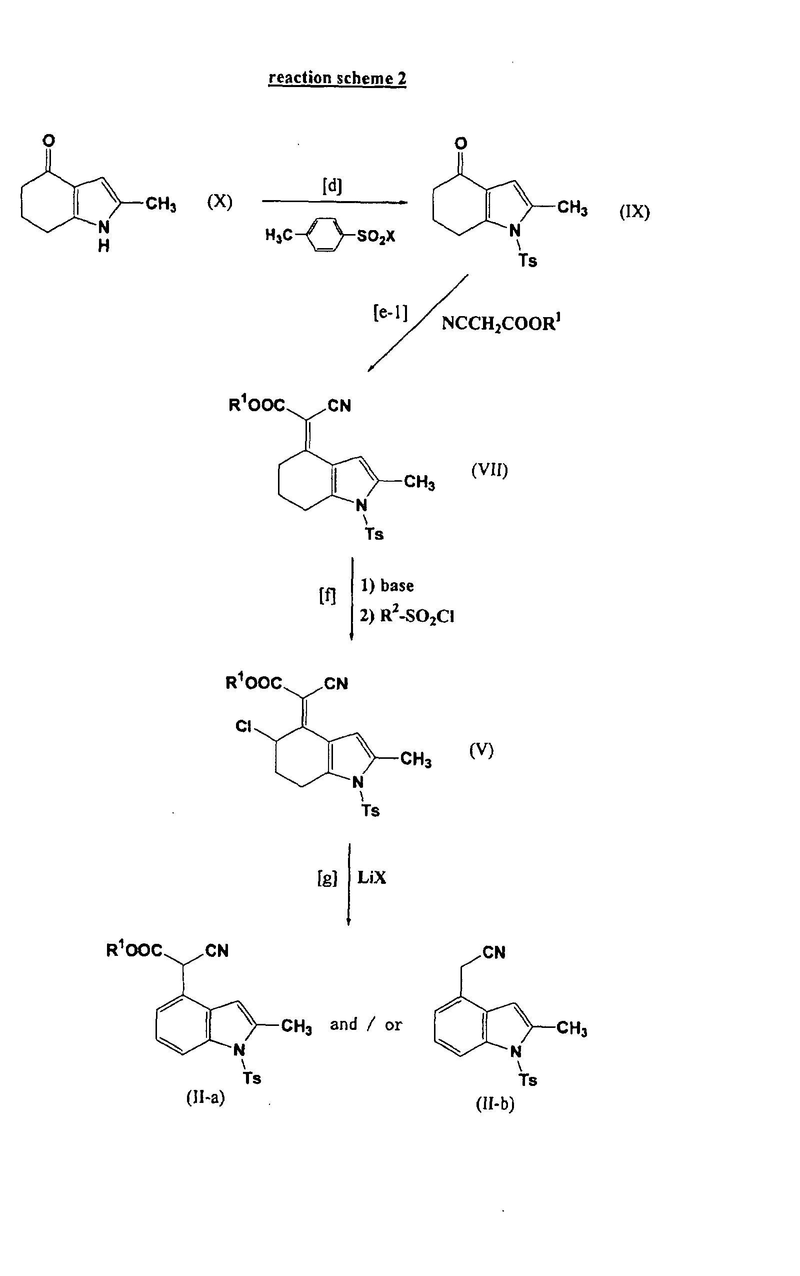

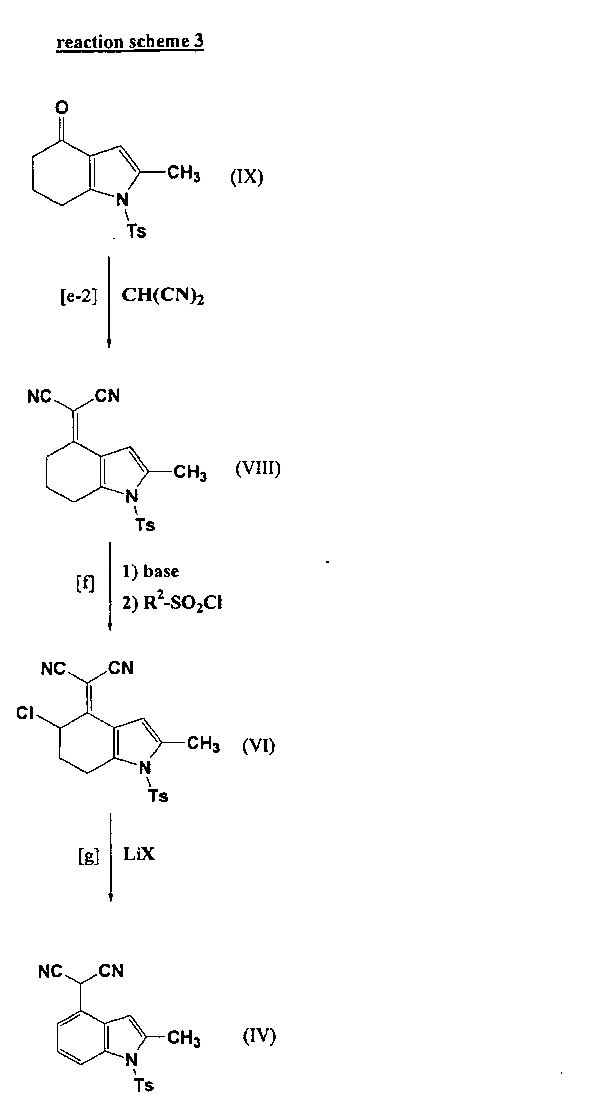

In the reaction scheme 2 and 3, X represents halogen atom, R¹ represents C1-4 alkyl, R² represents trifluoromethyl, phenyl or phenyl substituted by methyl, chloro or nitro at p-position.

Also, for example, the compound represented by the formula (X), which is the starting material in reaction scheme 2, can be prepared according to reaction scheme 4. In the scheme, X represents halogen atom.

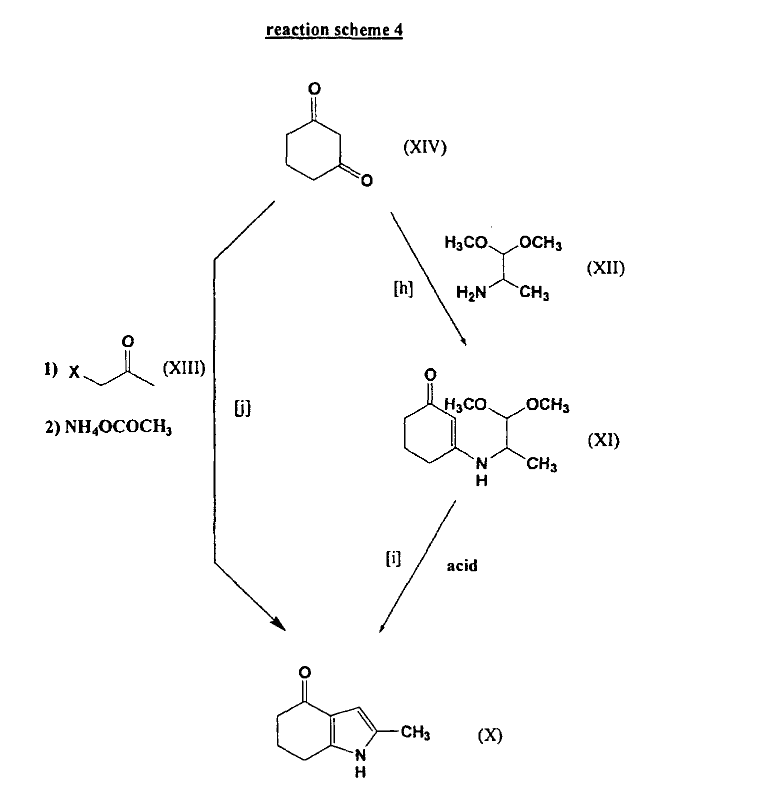

In the present invention, halogen atom is, chloride, bromide, iodide and fluoride.

In the present invention, C1-4 alkyl is methyl, ethyl, propyl, butyl and isomers thereof.

In the reaction scheme 1, the reaction of step [a] is a hydrolysis reaction under alkali condition, for example, it is carried out in a water-miscible organic solvent (e.g. ethanol, methanol, isopropanol, ethyleneglycol dimethyl ether, tetrahydrofuran (THF) or a mixture thereof), using an aqueous solution of an alkali (e.g. sodium hydroxide, potassium hydroxide) at reflux temperature of the solvent.

The reaction of step [b] is a decarboxylation reaction, for example, it is carried out in a solvent (e.g. acetic acid, water or a mixture thereof), using an acid (e.g. acetic acid, sulfuric acid, hydrochloric acid) at 25 ∼ 150 °C.

The reaction of step [c] is a hydrolysis reaction under alkali condition, for example, it is carried out in a water-miscible organic solvent (e.g. ethanol, methanol, isopropanol, ethyleneglycol dimethyl ether, tetrahydrofuran (THF) or a mixture thereof), using an aqueous solution of an alkali (e.g. sodium hydroxide, potassium hydroxide) at reflux temperature of the solvent.

In the reaction scheme 2, the reaction of step [d] is a tosylation reaction, for example, it is carried out in an organic solvent (e.g. acetonitrile, THF, ethyleneglycol dimethyl ether, diglyme), in the presence of a quaternary ammonium salt (e.g. tetrabutylammonium bromide) and an aqueous solution of an alkali (e.g. sodium hydroxide, potassium hydroxide) using tosyl halide (e.g. tosyl chloride, tosyl fluoride) at 0∼50 °C.

The reaction of step [e-1] is a carbon elongation reaction, for example, [e-1-a] it is carried out using Dean Stark equipment, in an organic solvent (e.g. toluene, xylene), in the presence of ammonium acetate and an organic acid (e.g. acetic acid, propionic acid), using cyanoacetic acid ester (e.g. methyl cyanoacetate, ethyl cyanoacetate) at reflux temperature of the solvent, or [e-1-b] it is carried out using Dean Stark equipment, in an organic solvent (e.g. toluene, xylene, benzene), in the presence of primary amine (e.g. cyclohexylamine, n-octyl amine) and an organic acid (e.g. acetic acid, propionic acid), using cyanoacetic acid ester (e.g. methyl cyanoacetate, ethyl cyanoacetate) at reflux temperature of the solvent.

According to the step [e-1-a] or [e-1-b] of the present invention, 2-cyano-2-[2-methyl-1-tosyl-5,6,7-trihydroindol-4-ylidene]acetic acid ester can be produced in high yield using cyanoacetic acid ester, which is very cheap reagent.

In the reaction scheme 3, the reaction of step [e-2] is a carbon elongation reaction, for example, [e-2-a] it is carried out using Dean Stark equipment, in an organic solvent (e.g. toluene, xylene), in the presence of ammonium acetate and an organic acid (e.g. acetic acid, propionic acid), using malononitrile at reflux temperature of the solvent, or [e-2-b] it is carried out using Dean Stark equipment, in an organic solvent (e.g. toluene, xylene, benzene), in the presence of primary amine (e.g. cyclohexylamine, n-octyl amine) and an organic acid (e.g. acetic acid, propionic acid), using malononitrile at reflux temperature of the solvent.

According to the step [e-2-a] or [e-2-b] of the present invention, 2-[2-methyl-1-tosyl-5,6,7-trihydroindol-4-ylidene]molononitrile can be produced in high yield using malononitrile, which is very cheap reagent.

In the reaction scheme 2 and 3, the reaction of step [f] is a halogenation reaction, for example, it is carried out in an organic solvent (e.g. THF, dimethylformamide (DMF)), using base (e.g. sodium hydride, lithium diisopropylamide, sodium t-butoxide, potassium t-butoxide) at 20 ∼ 55 °C, followed by using sulfonyl chloride (preferably cooled; e.g. benzene sulfonyl chloride, p-toluenesulfonyl chloride, p-chlorobenzenesulfonyl chloride, p-nitrobenzenesulfonyl chloride, trifluoromethanesulfonyl chloride) at -78 ∼ 30 °C.

According to the step [f] of the present invention, the compounds represented by the formula (V) and (VI), which is chlorinated at γ position, can be produced in high yield and γ-high selectivity against the chlorinated compounds at α position (the selectivity of γ position : α position is 99 : 1 or more), under mild conditions suitable for industrial production.

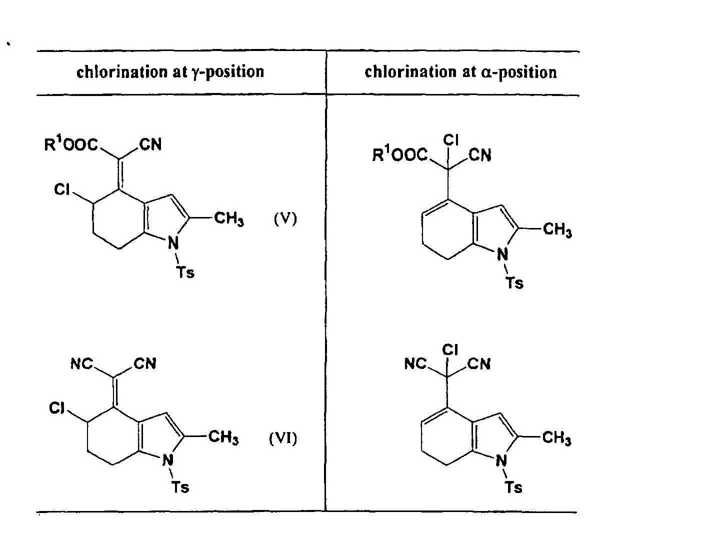

In the reaction scheme 2 and 3, the reaction of step [g] is an aromatization reaction, for example, it is carried out in an organic solvent (e.g. DMF, dimethylimidazolidinone, dimethylacetoamide, dimethylsulfoxide), using lithium halide (e.g. lithium bromide, lithium chloride) or hydrate thereof at 80 ∼ 120 °C. In the reaction scheme 4, the reaction of step [h] is a synthesis of enamine, for example, it is carried out in an organic solvent (e.g. acetonitrile, THF, ethylenegylcol dimethyl ether, diglyme, isopropanol), using 2-aminopropionaldehyde dimethyl acetal at reflux temperature of the solvent.

The reaction of step [i] is a cyclization reaction, for example, it is carried out in an aqueous solution of an acid (preferably keeping at over 65 °C;e.g. p-toluenesulfonic acid, hydrochloric acid), adding the solution of the compound prepared in step [h] in an organic solvent (e.g. acetonitrile, THF, ethyleneglycol dimethyl ether, diglyme, isopropanol) with keeping the temperature of the resulting mixture at over 65 °C (preferably keeping at over 70 °C).

According to the step [h] and [i] of the present invention, 4-oxo-2-methyl-4,5,6,7-tetrahydroindole represented by formula (X) can be produced in high yield.

The reaction of step [j] is (1) an introduction of an acetylmethyl group, and following (2) cyclization reaction. An introduction of an acetylmethyl group, for example, it is carried out in a water-miscible organic solvent (e.g. methanol, ethanol, isopropanol, acetonitrile), using a base (e.g. potassium hydroxide, sodium hydroxide, triton B), between room temperature and the reflux temperature of the solvent for 5 to 10 hours. Then, cyclization reaction is carried out. The cyclization reaction is carried out in a water-miscible organic solvent (e.g. methanol, ethanol, isopropanol, acetonitrile), using an ammonium acetate, at room temperature to the reflux temperature of the solvent for 1 to 5 hours.

According to the present invention, the compound represented by the formula (X) can be produced in one-pot from the compound represented by the formula (XIV) without isolation process in each step.

Each reaction may be preferably carried out in an atmosphere of inert gas (e.g. argon).

In each reaction step, the compounds using as starting material are known per se, or may be prepared by conventional known method from known compound. Also, the reagents in the present invention are known per se or may be prepared by known method.

In each reaction in the present specification, reaction products may be purified by conventional purification techniques, e.g. by distillation under atmospheric or reduced pressure, by high performance liquid chromatography, by thin layer chromatography or by column chromatography using silica gel or magnesium silicate; or by washing or by recrystallization. Purification may be carried out after each reaction or after a series of reactions.

The process for the preparation of indole-4-acetic acid, which is similar to 2-methylindole-4-acetic acid represented by the formula (I) in the present invention, is published in (1) J. Org. Chem., 44(22), 4003-4005 (1979) and (2) Hererocycles, 24(9), 2611-2618 (1986) hereinbefore described.

But, the starting materials and the reaction process in the present invention is completely different from that of the method described in (1). As for the method described in (2), expensive reagent is used in first step, and it is carried out under high temperature condition in second step. These methods are not suitable for industrial production. So, the methods described in these literatures do not suggest the process for the preparation of the present invention, that is suitable for industrial production using inexpensive reagents under mild conditions.

The process for the preparation of 4-oxo-2-methyl-4,5,6,7-tetrahydroindole represented by the formula (X) in the present invention is published in (3) the specification of US4868315, (4) Justus Liebigs Ann. Chem., 655, 20-26 (1952) and (5) the specification of JP2-15058 hereinbefore described.

But as for the method described in (3), reverse-extraction, which is complicated operation, is necessary. In the reaction scheme, the compound has to be isolated. Moreover, in first and second steps, the reaction takes 2 or 3 days. These methods are not suitable for industrial production. On the other hand, as for the method of the present invention, the reaction can be carried out between room temperature and the reflux temperature of the solvent. After the reaction was completed, the desired compound can be taken without complicated operation. And the starting material described in (4), is different from that of the present invention. Ammonia is used at the reaction step, and this reaction is carried out under high temperature. So, the reaction must be carried out under heating in a closed equipment such as autoclave, and it is irrelevance for industrial production. On the other hand, at the step [j] of the present invention, special equipment is unnecessary, because of using ammonium acetate between room temperature and the reflux temperature of the solvent. As for the method described in (5), the starting material differs from them of the present invention. In the present invention, an isolation process of starting material described in (5) is unnecessary.

As mentioned above, the step [j] of the present invention is superior to any of the related arts.

The process for the preparation of the compound represented by the formula (X), which is similar to 4-oxo-2-methyl-4,5,6,7-tetrahydroindole in the present invention, is published in (6) J. Org. Chem., 43(18), 3541-3544 (1978) hereinbefore described. But the yields of the compound 1 to compound 3, in which both of R and R' represent hydrogen atom, are low (33 %). On the other hand, in the present invention, the desired compound can be obtained in high yield by changing an order of adding the compound, i.e., adding the compound represented by the formula (XI) to p-toluenesulfonic acid and adjusting reaction temperature.

When the halogenation reaction described in (7) Hererocycles, 23(1), 165-170 (1985) hereinbefore described applied to the compound represented by the formula (VII) and (VIII), which is an intermediate of the compound in the present invention, the reaction does not proceed at all. The desired compound represented by the formula (V) or (VI) is not obtained. Moreover, it is reported that chlorinated by-product on 2-position of indole-ring and chlorinated desired compound on 5-posotion are obtained in (7). The selectivity of chlorination on desired 5-position is 86 % (table 2). On the other hand, according to the process for the preparation in this invention, the target compound chlorinated at γ position can be produced in high yield and high γ-selectivity (the selectivity of γ position : α position is 99 : 1 or more). This metod is more excellent than that of (7).

Thus, the synthetic route and reaction condition from the compound represented by the formula (XIV) to the compound represented by the formula (I) are novel and found out by these inventors for the first time. And 2-methylindole-4-acetic acid represented by the formula (I) can be produced in high yield using inexpensive reagents under mild conditions which are suitable for industrial production without special equipment. The novel compound prepared by the method, which is represented by the formula (I), is used as an key intermediate for medicines.

### Best Mode for Carrying Out the Invention

The following examples illustrate the present invention, but do not limit the present invention.

### Example 1(1)

### 4-Oxo-2-methyl-4,5,6,7-tetrahydroindole

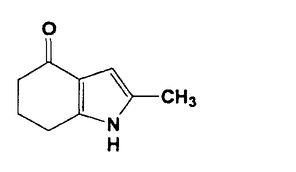

Cyclohexan-1,3-dione (1.00 g) was dissolved in 1.7M solution of potassium hydroxide in methanol (85% potassium hydroxide (5.61 g) in methanol (50 ml); 4.9 ml). To the solution was added chloroacetone (0.93 ml) and the mixture was refluxed for 7 hours. The reaction mixture was cooled to room temperature. To the mixture was added ammonium acetate (756 mg) and methanol (4.5 ml), the mixture was refluxed for 2 hours. The reaction mixture was cooled to room temperature, adjusted the pH to 6-7 with 4M aqueous solution of sodium hydroxide (1.7 ml), and then concentrated. To the residue was added dimethoxyethane (10 ml), and then an insoluble was removed by filtration. The filtrate was concentated and the residue was purified by column chromatography on silica gel (ethyl acetate : hexane = 1 : 1 → ethyl acetate : hexane : methanol = 5 : 5 : 1) to give the title compound (961 mg, 72.3 %) having the following physical data.
TLC : Rf 0.85 (methanol : ethyl acetate =1 : 3);
NMR (200 MHz, CDCl₃): δ 8.64 (brs, 1H), 6.18-6.16 (m, 1H), 2.76 (t, J = 6.0 Hz, 2H), 2.48-2.41 (m, 2H), 2.23 (s, 3H), 2.18-2.09 (m, 2H);
MS (FAV, Pos.):172, 150, 136;
IR (KBr): 3155, 1621, 1479, 1411, 1302, 1264, 1205, 1181, 1149, 1128, 1047, 1014, 992, 899, 820, 740, 718, 627, 600, 575, 543, 418 cm⁻¹;
m.p.: 202.5-204.0 °C.

### Example 1 (2)

### 4-Oxo-2-methyl-4,5,6,7-tetrahydroindole

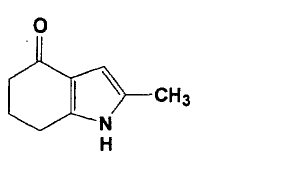

Cyclohexan-1,3-dione (1.00 g) was dissolved in 1.7M solution of potassium hydroxide in methanol (85% potassium hydroxide (5.61 g) in methanol (50 ml); 4.9 ml). To the solution was added chloroacetone (0.93 ml) and the mixture was refluxed for 7 hours. The reaction mixture was cooled to room temperature. To the mixture was added ammonium acetate (756 mg) and methanol (4.5 ml), the mixture was refluxed for 2 hours. The reaction mixture was cooled to room temperature, added water (6 ml), and then concentrated. The obtained crystal was washed with water (4 ml, twice) and dried to give the title compound (680 mg, 51.1 %) having the following physical data.
TLC : Rf 0.85 (methanol : ethyl acetate =1 : 3);
NMR (200 MHz, CDCl₃): δ 8.64 (brs, 1H), 6.18-6.16 (m, 1H), 2.76 (t, J = 6.0 Hz, 2H), 2.48-2.41 (m, 2H), 2.23 (s, 3H), 2.18-2.09 (m, 2H);
MS (FAV, Pos.):172, 150, 136;
IR (KBr): 3155, 1621, 1479, 1411, 1302, 1264, 1205, 1181, 1149, 1128, 1047, 1014, 992, 899, 820, 740, 718, 627, 600, 575, 543, 418 cm⁻¹;
m.p.: 202.5-204.0 °C.

### Example 2

### 1-(3-Dimethoxypropyl-2-yl)amino-3-cyclohexenone

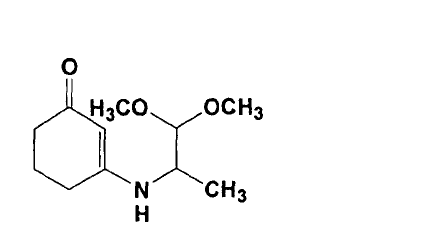

To a vessel were added 1,3-cyclohexanedione (5.00 g) and acetonitrile (24 ml) at 18 °C under an argon atmosphere. To the mixture was added 2-aminopropionaldehyde dimethylacetal (5.58 g) during 2 minutes at room temperature with stirring. After the addition was completed, the solution was stirred for 3 hours over 80 °C (internal temperature). The reaction mixture was left overnight and the title compound was obtained having the following physical data.
TLC : Rf 0.50 (methanol : ethyl acetate =1 : 3);
NMR (200 MHz, CDCl₃): δ 5.14 (s, 1H), 4.65-4.62 (m, 1H), 4.23 (d, J = 3.2 Hz, 1H), 3.67-3.58 (m, 1H), 3.45 (s, 3H), 3.43 (s, 3H), 2.35-2.28 (m, 4H), 2.02-1.93 (m, 2H), 1.15 (d, J = 6.8 Hz, 3H);
MS (FAV, Pos.):236, 214, 182, 176, 154, 136;
IR (Liquid film):3255, 3064, 2941, 2834, 1738, 1669, 1552, 1455, 1432, 1369, 1319, 1257, 1191, 1134, 1067, 981, 968, 950, 937, 908, 859, 809, 764, 672, 651, 610, 557, 535, 511, 468 cm⁻¹.

### Example 3 (1)

### 4-Oxo-2-methyl-4,5,6,7-tetrahydroindole

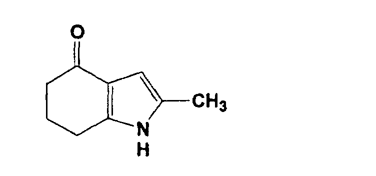

To a 2M aqueous solution of p-toluenesulfonic acid (33.5 ml) at an internal temperature of ca. 80 °C was added dropwise the compound prepared in example 2 at an internal temperature of ca. 70 °C while keeping the internal temperature of the mixture over 73 °C. The mixture was stirred for 2 hours at an internal temperature of ca. 80 °C. The reaction mixture was cooled to 25 °C, adjusted the pH to 11 with 4M aqueous solution of sodium hydroxide, and then kept standing overnight. The solution was concentrated and then azeotroped with toluene. The residue was purified by column chromatography on silica gel (hexane : ethyl acetate =1 : 2) to give the title compound (4.78 g, 71.9 %) having the following physical data.
TLC : Rf 0.85 (methanol : ethyl acetate =1 : 3);
NMR (200 MHz, CDCl₃): δ 8.64 (brs, 1H), 6.18-6.16 (m, 1H), 2.76 (t, J = 6.0 Hz, 2H), 2.48-2.41 (m, 2H), 2.23 (s, 3H), 2.18-2.09 (m, 2H);
MS (FAV, Pos.):172, 150, 136;
IR (KBr): 3155, 1621, 1479, 1411, 1302, 1264, 1205, 1181, 1149, 1128, 1047, 1014, 992, 899, 820, 740, 718, 627, 600, 575, 543, 418 cm⁻¹;
m.p.: 202.5-204.0 °C.

### Example 3 (2)

### 4-Oxo-2-methyl-4,5,6,7-tetrahydroindole

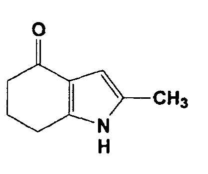

To a 2M aqueous solution of p-toluenesulfonic acid (33.5 ml) at an internal temperature of ca. 80 °C was added dropwise the compound prepared in example 2 at an internal temperature of ca. 70 °C while keeping the internal temperature of the mixture over 73 °C. The mixture was stirred for 2 hours at an internal temperature of ca. 80 °C. The reaction mixture was left for a while, adjusted the pH to 11 with 4M aqueous solution of sodium hydroxide. The 40% volumes of the reaction mixture was removed. The residue was stirred for 1 hour at room temperature, and then the appeared crystal was collected by filtration. The crystal was washed with water, and dried under reduced pressure for 15 hours at 40 °C to give the title compound (3.73 g, 56 %) having the following physical data.
TLC : Rf 0.85 (methanol : ethyl acetate =1 : 3);
NMR (200 MHz, CDCl₃): δ 8.64 (brs, 1H), 6.18-6.16 (m, 1H), 2.76 (t, J = 6.0 Hz, 2H), 2.48-2.41 (m, 2H), 2.23 (s, 3H), 2.18-2.09 (m, 2H);
MS (FAV, Pos.):172, 150, 136;
IR (KBr): 3155, 1621, 1479, 1411, 1302, 1264, 1205, 1181, 1149, 1128, 1047, 1014, 992, 899, 820, 740, 718, 627, 600, 575, 543, 418 cm⁻¹;
m.p. : 202.5-204.0 °C.

### Example 4

### 2-Methyl-4-oxo-1-tosyl-4,5,6,7-tetrahydroindole

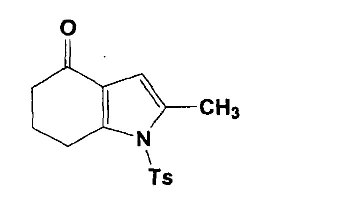

The compound prepared in example 3 (2) (111 g) was suspended in acetonitrile (1110 ml). To the suspension were added tetrabutylammonium bromide (72 g) and 50 % aqueous solution of sodium hydroxide (596 ml) at room temperature. To the suspension was added dropwise a solution of tosylchloride (284 g) in THF (555 ml) at room temperature, and the mixture was stirred for 1 hour. To the water (6700 ml) was added the reaction solution, and the mixture was stirred for 30 minutes at 15 °C. The appeared crystal was collected by filtration, washed with water (1200 ml, twice) and dried under reduced pressure for 15 hours at 40 °C to give the title compound (219 g, 97.0 %) having the following physical data.
TLC : Rf 0.75 (hexane : ethyl acetate =1 : 1);
NMR (200 MHz, CDCl₃): δ 7.65 (d, J = 8.2 Hz, 2H), 7.34 (d, J = 8.2 Hz, 2H), 6.30 (s, 1H), 3.12 (t, J = 6.0 Hz, 2H), 2.44-2.39 (m, 8H), 2.18-2.05 (m, 2H);
MS (FAB, Pos.):326, 304, 176;
IR (KBr): 3437, 2952, 1672, 1596, 1540, 1493, 1459, 1434, 1415, 1366, 1304, 1258, 1238, 1215, 1193, 1166, 1131, 1094, 1052, 992, 902, 835, 811, 782, 729, 701, 678, 653, 605, 585 cm⁻¹.

### Example 5

### Ethyl 2-cyano-2-[2-methyl-1-tosyl-5,6,7-trihydroindol-4-ylidene]acetate

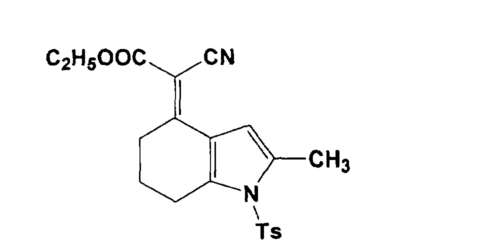

The compound prepared in example 4 (199 g) was dissolved in toluene (1990 ml) at 14 °C. To the solution were added ammonium acetate (55.6 g), acetic acid (157.5 g) at 16 °C, and ethyl cyanoacetate (111.8 g). The mixture was refluxed for 19 hours while removing water using Dean Stark equipment. The reaction solution was concentrated and then azeotroped with toluene. To the residue was added isopropanol (1990 ml) and water (995 ml) and the mixture was dissolved by heating. After cooling at ambient temperature, the mixture was cooled to an internal temperature of 0 °C and stirred for 20 minutes. The appeared crystal was collected by filtration. The crystal was washed with isopropaanol / water (900 ml / 900 ml, twice) and dried for 13 hours at 60 °C under reduced pressure to give the title compound (192 g, 74.3 %) having the following physical data.
TLC : Rf 0.63 (toluene : ethyl acetate =9 : 1);
NMR (75 MHz, CDCl₃): δ 7.64 (d, J = 8.3 Hz, 2H), 7.34 (d, J = 8.3 Hz, 2H), 7.15-7.10 (m, 1H), 4.27 (q, J = 7.2 Hz, 2H), 3.18-2.98 (m, 4H), 2.44 (s, 3H), 2.43 (s, 3H), 2.00-1.80 (m, 2H), 1.35 (t, J = 7.2 Hz, 3H);
MS (FAB, Pos.):421, 399, 391, 353, 329, 307;
IR (KBr): 3754, 3678, 3449, 2934, 2216, 1714, 1655, 1596, 1536, 1413, 1373, 1349, 1333, 1243, 1221, 1193, 1166, 1091, 1059, 949, 810, 774, 704, 686, 652, 591, 544, 429 cm⁻¹;
m.p.: 124.1-125.9 °C.

### Example 5(1)

### 2-[2-Methyl-1-tosyl-5,6,7-trihydroindol-4-ylidene]malononitrile

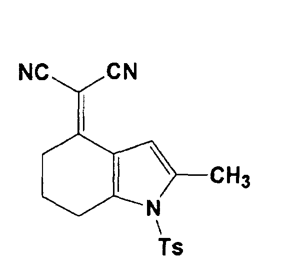

The compound having the following physical data was obtained according to the same procedure as described example 5, using malononitrile instead of ethyl cyanoacetate.
TLC : Rf0.82 (hexane : ethyl acetate = 1 : 1);
NMR (200 MHz, CDCl₃): δ 7.66 (2H, d, J = 8.5Hz), 7.40 (2H, d, J = 8.5Hz), 6.96-6.90 (1H, m), 3.11 (3H, t, J = 6.2 Hz), 2.80 (3H, t, J = 6.2Hz), 2.46 (3H, s), 2.43 (3H, s), 2.10-1.90 (2H, m).

### Example 6

### Ethyl 2-cyano-2-[2-methyl-1-tosyl-5,6,7-trihydroindol-4-ylidine]acetate

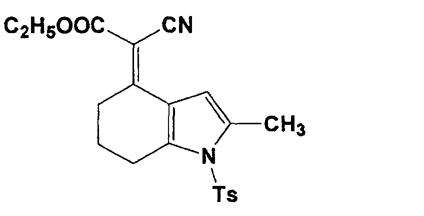

The solution of the compound prepared in example 4 (80 g), ethyl cyanoacetate (35.8 g), cyclohexylamine (5.23g) and propionic acid (5.86 g) in toluene (400 ml) was refluxed for 6 hours using Dean Stark equipment. The reaction solution was cooled and concentrated. To the residue was added isopropanol (1360 ml) and the mixture was dissolved by heating. The solution was cooled and stirred for 14 hours at room temperature. To the solution was added water. The mixture was cooled at 0 °C and then stirred for 1 hour. The appeared crystal was collected by filtration, washed with isopropanol and dried to give the title compound (89.6 g, 85.3 %) having the following physical data.
TLC : Rf 0.63 (toluene : ethyl acetate =9 : 1);
NMR (75 MHz, CDCl₃): δ 7.64 (d, J = 8.3 Hz, 2H), 7.34 (d, J = 8.3 Hz, 2H), 7.15-7.10 (m, 1H), 4.27 (q, J = 7.2 Hz, 2H), 3.18-2.98 (m, 4H), 2.44 (s, 3H), 2.43 (s, 3H), 2.00-1.80 (m, 2H), 1.35 (t, J = 7.2 Hz, 3H);
MS (FAB, Pos.):421, 399, 391, 353, 329, 307;
IR (KBr): 3754, 3678, 3449, 2934, 2216, 1714, 1655, 1596, 1536, 1413, 1373, 1349, 1333, 1243, 1221, 1193, 1166, 1091, 1059, 949, 810, 774, 704, 686, 652, 591, 544, 429 cm⁻¹;
m.p. : 124.1-125.9 °C.

### Example 7

### Ethyl 2-cyano-2-[5-chloro-2-methyl-1-tosyl-5,6,7-trihydroindol-4-ylidene]acetate

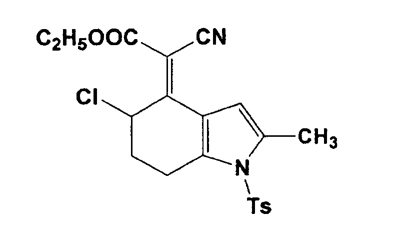

A sodium hydride (60 ∼ 72 wt%; 10.6 g) was suspended in THF (150 ml) under an argon atmosphere and the mixture was stirred at an internal temperature of 40 ∼ 50 °C. To the suspension was added dropwise a solution of the compound prepared in example 5 (100.4 g) in THF (400 ml) and the mixture was stirred for 1 hour at an internal temperature of 45 ∼ 53 °C. The reaction solution was cooled to room temperature.

A solution of benzenesulfonyl chloride (46.7 g) in THF (150 ml) was cooled at an internal temperature of -12 ∼ -10 °C. To the solution was added dropwise the above mentioned reaction solution below an internal temperature of 5 °C. The mixture solution was stirred for 30 minutes at -10 °C. To an aqueous solution of ammonium chloride (130 g / 600 ml) was added the reaction solution, and then the mixture was extracted with t-butyl methyl ether (500 ml). The organic layer was concentrated to give the title compound (128.2 g) having the following physical data. The obtained compound was used for the next step without further purification.
TLC : Rf 0.68 (toluene : ethyl acetate =9 : 1);
NMR (200 MHz, CDCl₃): δ 7.64 (d, J = 8 Hz, 2H), 7.35 (d, J = 8 Hz, 2H), 7.11 (d, J = 1 Hz, 1H), 6.42 (dd, J = 3.6, 2.8 Hz, 1H), 4.33 (q, J = 7 Hz, 2H), 3.35-3.20 (m, 2H), 2.44 (s, 3H), 2.41 (s, 3H), 2.50-2.30 (m, 1H), 2.25-2.00 (m, 1H), 1.37 (t, J = 7 Hz, 3H);
MS (EI, Pos.):432, 397, 387, 369, 323, 277, 241;
IR (KBr): 3823, 2678, 3412, 2980, 2935, 2216, 1723, 1596, 1566, 1492, 1475, 1428, 1373, 1347, 1247, 1191, 1159, 1089, 1057, 1028, 1005, 942, 803, 781, 756, 703, 686, 674, 649, 598 cm⁻¹;
m.p.: 101.5-104.2 °C.

### Example 7(1)

### 2-[2-Methyl-1-tosyl-5-chloro-5,6,7-trihydroindol-4-ylidene]malononitrile

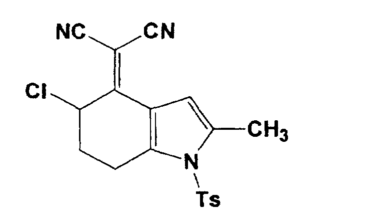

The compound having the following physical data was obtained according to the same procedure as described example 7, using the compound prepared in example 5 (1).
TLC: Rf 0.44 (hexane : ethyl acetate = 2 : 1);
NMR (200 MHz, CDCl₃): δ 7.67 (2H, d, J = 8.5Hz), 7.38 (2H, d, J = 8.5Hz), 6.96-6.90 (1H, m), 5.24 - 5.08 (1H, m), 3.50 - 3.00 (2H, m), 2.46 (3H, s), 2.42 (3H, s), 2.60-2.00 (2H, m).

### Example 8

### Ethyl 2-cyano-2-[2-methyl-1-tosyl-indol-4-yl]acetate (8(a)) and 4-cyanomethyl-2-methyl-1-tosylindole (8(b))

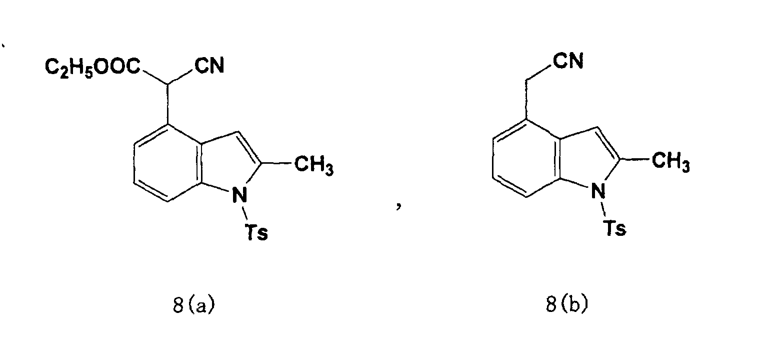

The compound prepared in example 7 (118.8 g) in DMF (470 mL) was dissolved by heating. To the solution was added lithium bromide monohydrate (24.5 g) and the mixture was stirred for 3 hours at an internal temperature of 100 ∼ 105 °C. After cooling to room temperature, the mixture was added to water (940 ml) and then extracted with ethyl acetate (940 ml). The aqueous layer was extracted with ethyl acetate (940 ml) again. The combined organic layer was washed with an aqueous solution of sodium bicarbonate (19.7 g / 470 ml) and concentrated to give the title compound (compound 8(a) and compound 8(b)) as mixture (105.7 g) having the following physical data. The obtained mixture was used for the next step without further purification.
compound 8(a)
TLC : Rf0.49 (toluene : ethyl acetate =9 : 1);
NMR (200 MHz, CDCl₃): δ 8.30-8.10 (m, 1H), 7.68 (d, J = 8.2 Hz, 2H), 7.40-7.10 (m, 4H), 6.54 (s, 1H), 4.89 (s, 1H), 4.21 (dq, J = 7, 1.4 Hz, 2H), 2.64 (s, 3H), 2.37 (s, 3H), 1.22 (t, J = 7 Hz, 3H);
MS (FAB, Pos.):397, 391, 323, 307, 289;
IR (Liquid film):3057, 2983, 2930, 1747, 1598, 1572, 1494, 1432, 1390, 1370, 1307, 1293, 1256, 1221, 1189, 1178, 1159, 1122, 1099, 1068, 1029, 1017, 925, 880, 856, 813, 777, 746, 705, 677 cm⁻¹.
compound 8 (b)
TLC : Rf0.4 (toluene : ethyl acetate = 9 : 1);
NMR (200 MHz, CDCl₃): δ 8.16 (d, J = 7.6 Hz, 1H), 7.67 (d, J = 8.4 Hz, 2H), 7.32-7.15 (m, 4H), 6.44-6.36 (m, 1H), 3.83 (s, 2H), 2.64 (s, 3H), 2.36 (s, 3H);
MS (FAB, Pos.):347, 325;
IR (KBr): 3404, 3093, 2930, 2250, 1701, 1655, 1597, 1571, 1492, 1431, 1407, 1370, 1297, 1258, 1221, 1176, 1098, 1062, 1041, 1011, 933, 805, 777, 743, 704, 683, 645, 585, 561, 542 cm⁻¹.

### Example 9

### 2-Methylindole-4-acetic acid

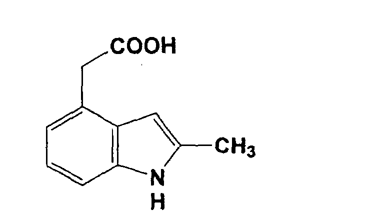

To the mixture prepared in example 8 (97.7 g) was added ethanol (300 ml) and an aqueous solution of potassium hydroxide (121 g / 490 ml) and the mixture was refluxed. After cooling to room temperature, the mixture was concentrated. To the residue was added water (430 ml) and the mixture was washed with t-butyl methyl ether (430 ml). The aqueous layer was washed with t-butyl methyl ether again. An insoluble substance was removed by filtration. The pH of filtrate was adjusted to 3 by concentrated hydrochloric acid under ice cooling. The solution was stirred for 30 minutes. The appeared crystal was collected by filtration, and washed with water (220 ml, twice). The crystal was dissolved in a mixture of isopropanol / water (122 ml / 368 ml) under heating, and then the mixture was cooled to room temperature. The solution was permitted to stand for 1 hour at 4 °C. The appeared crystal was collected by filtration, washed with a mixture of isopropanol / water (1 / 3, 80 ml, twice) and dried for 19 hours at 40 °C to give the title compound (30.6 g, 74.8 %) having the following physical data.
TLC : Rf 0.35 (Ethyl acetate);
NMR (200 MHz, DMSO-d₆): δ 11.0-10.8 (br, 1H), 7.15 (d, J = 7Hz, 1H), 6.91 (t, J = 7 Hz, 1H), 6.79 (d, J = 7 Hz, 1H), 6.16-6.08 (m, 1H), 3.67 (s, 2H), 3.62 (s, 3H);
MS (FAB, Pos.):190, 170, 144;
IR (KBr): 3855, 3823, 3678, 3652, 3631, 3405, 2906, 1702, 1617, 1557, 1509, 1439, 1405, 1348, 1295, 1278, 1253, 1214, 1178, 1056, 955, 760, 708, 633, 513, 483, 420 cm⁻¹;
m.p.: 199.1-202.4 °C.

### Example 10

### 2-[2-Methyl-1-tosylindol-4-yl]malononitrile

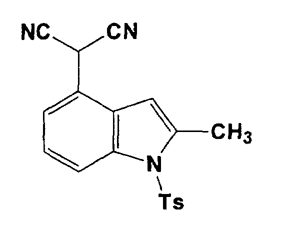

The title compound having the following physical data was obtained according to the same procedure as described example 8, using the compound prepared in example 7 (1).
TLC : Rf 0.33 (hexane : ethyl acetate =2 : 1);
NMR (200 MHz, CDCl₃): δ 8.40 - 8.20 (1H, m), 7.69 (2H, d, J = 8.5Hz), 7.40-7.25 (2H, m), 7.23 (2H, d, J = 8..5Hz), 6.55 - 6.45 (1H, m), 5.18 (1H, s), 2.66 (3H, s), 2.37 (3H, s).

### Example 11

### 2-[2-Methylindol-4-yl]malononitrile

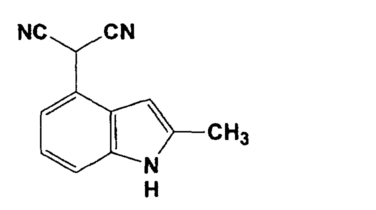

To the compound prepared in example 10 (232 mg) was added potassium hydroxide (740 mg), water (1.5 mL) and ethanol (0.8 ml) and the mixture was refluxed for 23 hours. After cooling, ice-water and 6M hydrochloric acid was added to the reaction mixture, and then the mixture was extracted with t-butyl methyl ether. The organic layer was dried over anhydrous magnesium sulfate and concentrated. The residue was purified by column chromatography on silica gel to give the title compound (108 mg, 83.8 %) having the following physical data
TLC: Rf 0.58 (toluene : ethyl acetate = 2 : 1);
NMR (200 MHz, CDCl₃): δ 8.25 (brs, 1H), 7.41-7.24 (m, 4H), 6.36 (s, 1H), 5.22 (s, 1H), 2.49 (s, 3H)

### Example 12

### 2-Methylindole-4-acetic acid

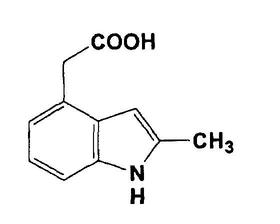

To the compound prepared in example 11 (48 mg) was added acetic acid (1 ml), water (1 ml) and concentrated sulfuric acid (1 ml), and the mixture was stirred for 4 hours at 110 °C. The reaction solution was cooled, poured into water and extracted with t-butyl methyl ether. The organic layer was dried over anhydrous magnesium sulfate and concentrated. The residue was purified by column chromatography on silica gel (hexane : ethyl acetate = 1 : 1) to give the title compound (33 mg, 70 %) having the following physical data.
TLC : Rf 0.35 (Ethyl acetate);
NMR (200 MHz, DMSO-d₆): δ 11.0-10.8 (br, 1H), 7.15 (d, J = 7 Hz, 1H), 6.91 (t, J = 7 Hz, 1H), 6.79 (d, J = 7 Hz, 1H), 6.16-6.08 (m, 1H), 3.67 (s, 2H), 3.62 (s, 3H);
MS (FAB, Pos.):190, 170, 144;
IR (KBr): 3855, 3823, 3678, 3652, 3631, 3405, 2906, 1702, 1617, 1557, 1509, 1439, 1405, 1348, 1295, 1278, 1253, 1214, 1178, 1056, 955, 760, 708, 633, 513, 483, 420 cm⁻¹;
m.p. : 199.1-202.4 °C.

## Claims

1. 2-Methylindole-4-acetic acid represented by formula (I):

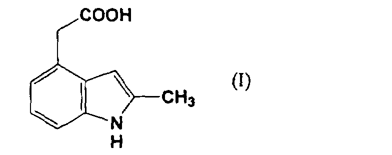

2. A process for producing the compound represented by formula (I): 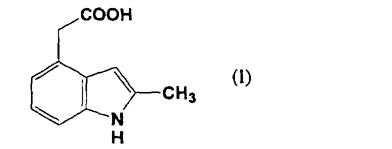 comprising subjecting a compound represented by formula (II-a): 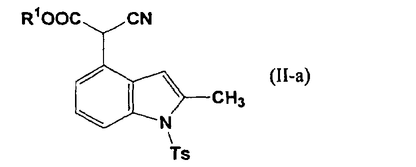 (wherein R¹ represents C1-4 alkyl; Ts represents tosyl group), a compound represented by formula (II-b): 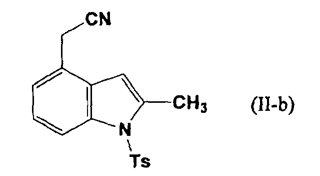 (wherein Ts represents tosyl group) or a mixture thereof to hydrolysis reaction under alkali condition.

3. A process for producing the compound represented by formula (I): 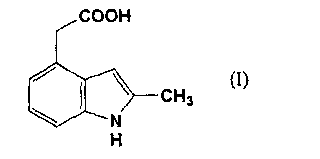 comprising reacting a compound represented by formula (III): 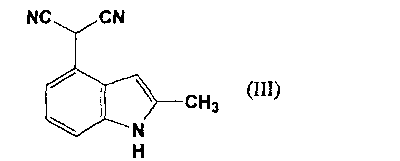 with an acid.

4. A process for producing the compound represented by formula 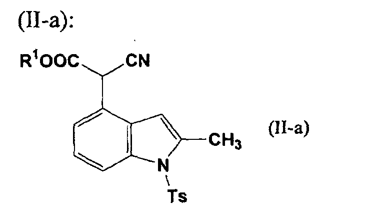 (wherein symbols in the formula have the same meanings as described above), the compound represented by formula (II-b): 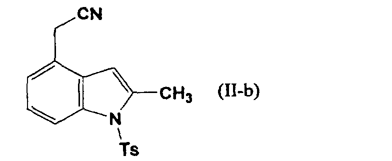 (wherein symbols in the formula have the same meanings as described above) or the mixture thereof according to claim 2, comprising subjecting a compound represented by formula (X): 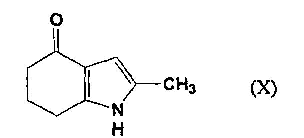 to a tosylation reaction, reacting the resulting compound represented by formula (IX): 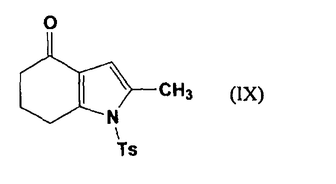 (wherein Ts represents tosyl group) with a cyanoacetate ester, subjecting a resulting compound represented by formula (VII): 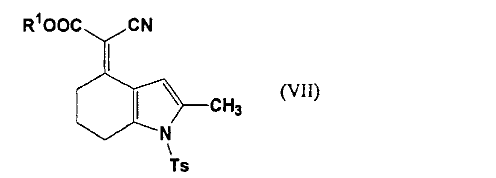 (wherein R¹ represents C1-4 alkyl, and Ts represents tosyl group), to a halogenation reaction, and reacting the resulting compound represented by formula (V): 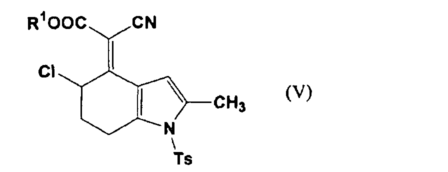 (wherein symbols in the formula have the same meanings as described above) with a lithium halide or hydrate thereof.

5. A process for producing the compound represented by formula (III): 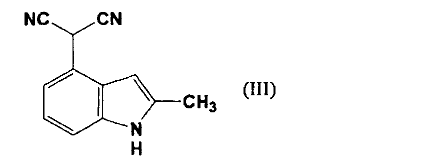 according to claim 3, comprising subjecting the compound represented by formula (X): 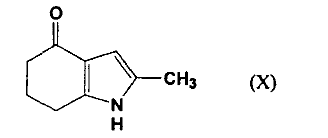 to a tosylation reaction, reacting the resulting compound represented by formula (IX): 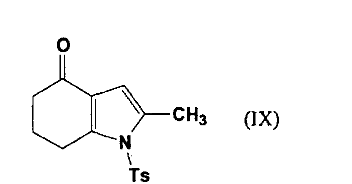 (wherein Ts represents tosyl group) with malononitrile, subjecting the resulting compound represented by formula (VIII): 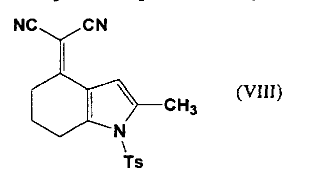 (wherein Ts represents tosyl group) to a halogenation reaction, reacting the resulting compound represented by formula (VI): 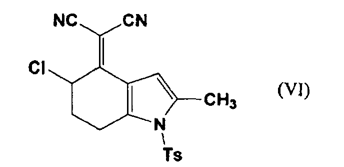 (wherein symbols in the formula have the same meanings as described above) with a lithium halide or hydrate thereof, and subjecting the resulting compound represented by formula (IV): 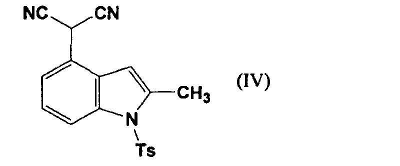 (wherein symbols in the formula have the same meanings as described above) to a hydrolysis reaction under alkali condition.

6. A process for producing the compound represented by formula (X): 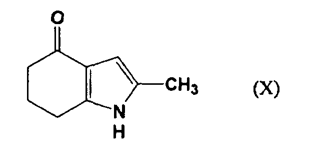 according to claim 4 or 5, comprising reacting a compound represented by formula (XIV): 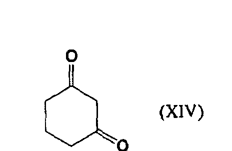 with represented by a compound represented by formula (XI): 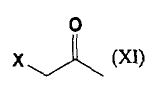 (wherein X represents halogen atom) between room temperature and a reflux temperature of a solvent for 5-10 hours, followed by reacting with ammonium acetate at room temperature to a reflux temperature of a solvent for 1-5 hours.

7. A process for producing the compound represented by formula (X): 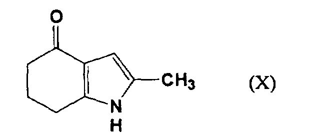 according to claim 4 or 5, comprising reacting the compound represented by formula (XIV): 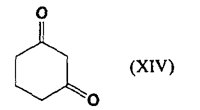 with a compound represented by formula (XIII): 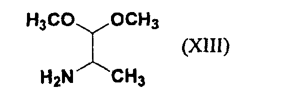 and adding the resulting compound represented by formula (XII): 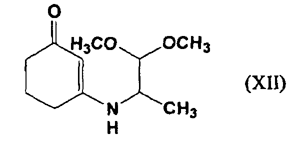 to an acid.
